# EUROPEAN PATENT SPECIFICATION

(11) **EP 4 321 120 B1**
(45) Date of publication and mention of the grant of the patent: **06.05.2026**
(21) Application number: 22794335.4
(22) Date of filing: 03.03.2022
(51) Int. Cl.: A61B 34/30, A61B 17/29

(54) **REAR-END TRANSMISSION DEVICE, MEDICAL INSTRUMENT, AND SURGICAL ROBOT**
HINTERE ÜBERTRAGUNGSVORRICHTUNG, MEDIZINISCHES INSTRUMENT UND CHIRURGISCHER ROBOTER
DISPOSITIF DE TRANSMISSION D'EXTRÉMITÉ ARRIÈRE, INSTRUMENT MÉDICAL ET ROBOT CHIRURGICAL

(30) Priority: 30.04.2021 CN 202110484481
(43) Date of publication of application: 14.02.2024
(73) Proprietor: Cornerstone Technology (Shenzhen) Limited, Shenzhen, Guandong 518066 (CN)
(72) Inventor: YIP, Hoiwut, Shenzhen, Guangdong 518066 (CN)
(74) Representative: Dennemeyer & Associates S.A.
(86) International application number: PCT/CN2022/078980
(87) International publication number: WO 2022/227856

(56) References cited:
- WO-A1-2010/009224
- WO-A1-2014/151952
- WO-A1-2020/252184
- CN-A- 102 143 719
- CN-A- 105 979 889
- CN-A- 106 470 632
- CN-A- 112 043 393
- CN-A- 112 367 928
- CN-A- 113 208 732
- US-A1- 2008 119 870
- US-A1- 2018 110 577
- US-A1- 2019 059 986
- US-A1- 2019 380 800
- US-A1- 2020 000 533

## Description

### TECHNICAL FIELD

The various embodiments described in this document relate in general to the technical field of medical devices, and more specifically to a rear-end transmission device, a medical device, and a surgical robot.

### BACKGROUND

In robot-assisted minimally invasive surgery, surgical instruments connected at the end of the robot enter the human body through wounds on the surface of the human body or natural canals of the human body, to be operated on tissues in the human body. The surgical instrument mainly includes an effector or tool (such as a pair of surgical forceps, a cutting tool, or a cauterizing tool) mounted to a wrist mechanism at a front end of the instrument, the wrist mechanism providing multiple degrees of freedom for movement of the front end, a main shaft extending from a rear end of the instrument to the front end, and a power and transmission device at the rear end of the instrument. The effector at the front end and the wrist mechanism are generally driven by multiple cables fixed thereto, and the cables run through the main shaft of the surgical instrument and are driven by the power and transmission device at the rear end.

For surgical forceps and other holding or cutting tools, the wrist mechanism is generally required to realize three degrees of freedom: pitch, yaw, and grip. Cooperated with extra degrees of freedom of the rear end of the robot, the wrist mechanism can realize the movement required to perform surgical operations. According to specific implementation of the wrist mechanism, the number of driving cables required for the wrist mechanism varies, for example, there are generally 4 or 6 cables configured for the wrist mechanism. In addition, in order to realize a large-scale movement (for example, rotation of -90° to 90°) of each joint of the wrist mechanism, it is necessary to arrange extra pulleys on the wrist mechanism to guide cables. However, addition of the extra pulleys may hinder the miniaturization of the front end of the surgical instrument, and the use of more cables may also increase the size and cost of the instrument.

The existing rear-end transmission device for driving the 4-cable wrist mechanism realizes release of two cables and pulling in of other two cables through swing of a connecting rod or a rocker arm, thereby realizing the pitch of the wrist mechanism. In this way, with the change of a pitch angle, a length of a pulled-in cable is not equal to a length of a released cable, which may lead to a change in tension of the cables, and then lead to problems such as reduced accuracy due to transmission error caused by loosened cables, or accelerated wear due to excessive frictional resistance caused by over-tensioned cables. To avoid such problems, one method is to limit a range of a rotation angle of a pitch joint, but this method may affect the function of the instrument. In addition, when using this mechanism, a rotation angle of an output terminal of the motor at the rear end has a nonlinear relationship with the pitch angle of the wrist mechanism, which requires extra calibration to achieve accurate position control and thus increases extra workload.

WO2010009224A1 relates to a transmission or backend mechanism for a medical instrument connects four cables to three motorized degrees of freedom. The transmission employs a first drive mechanism attached to first and second cables and a second drive mechanism attached to third and fourth cables, where each mechanism can include a capstan or a lever system that pulls in one cable while simultaneously feeding out another cables. A third drive mechanism has a first pivot about which a portion of the first drive mechanism rotates, a second pivot about which a portion of the second drive mechanism rotates, and a third pivot about which the third drive mechanism rotates. Rotation of the third drive mechanism about the third pivot pulls in at least one of the first and second cables and feeds out at least one of the third and fourth cables.

WO2020252184A1 relates to an actuation assembly for a medical instrument connects four bands to three motorized degrees of freedom. The actuation assembly includes a first actuator, a second actuator and a third actuator. The first actuator is coupled to a first band and a second band, the first actuator being operable to pull in one of the first and second bands and feed out the other of the first and second bands. The third actuator is coupled to a third band and a fourth band, the third actuator being operable to pull in one of the third and fourth bands and feed out the other of the third and fourth bands. Each of the first, second, third, and fourth bands pass through a second actuator, and the second actuator is operable to increase the travel path of both the first and second bands or both the third and fourth bands.

WO2014151952A1 relates to a surgical tool includes a tool shaft, and end effector and a wrist that couples the end effector to the tool shaft. The tool includes a drive mechanism configured to effect movement of one or both of the wrist and the end effector in yaw and pitch via independent actuation of four independent cable ends of two or more independent cables that extend between the drive mechanism and the wrist.

CN112043393A relates to a surgical instrument, a slave operation device applying the surgical instrument and a surgical robot with the slave operation device, the surgical instrument comprises an end effector, a driving device and cables, and the driving device is configured to drive the end effector to move through the mooring rope. The cables comprise a first pair of cables and a second pair of cables which are used for driving the end executor to execute yawing motion, and a third pair of cables which are used for driving the end executor to execute pitching motion, and due to the fact thatyawing motion is orthogonal to pitching motion, the third pair of cables have a coupling relation with the first pair of cables and the second pair of cables on the end executor. The driving device isprovided with a decoupling mechanism used for releasing the coupling relation, the decoupling mechanism comprises a main decoupling piece, an auxiliary decoupling piece and a decoupling cable, the main decoupling piece is connected with the auxiliary decoupling piece through the decoupling cable, and the main decoupling piece is used for coaxially rotating with a third driving unit and driving the auxiliary decoupling piece to slide relative to the body through the decoupling cable.

US20080119870A1 relates to a robotic surgical tool having opposing jaws, the working element of the robotic surgical tool is made of a different material from the drive element of the robotic surgical tool. The two elements may be manufactured independently and assembled together at a later stage. The material comprising each element may thus have properties more appropriate to the function each element plays in the robotic surgical tool. For example, the metal selected to comprise the blade of a surgical scissor may be corrosion resistant and capable of being sharpened to a high degree.

### SUMMARY

A series of simplified concepts have been introduced in this section, which will be further elaborated in the detailed description. The section of the disclosure does not mean attempting to limit key features and essential technical features of the claimed technical solution, nor does it mean attempting to determine the scope of protection of the claimed technical solution. The invention is set out in the appended set of claims.

Embodiments of the disclosure provide a rear-end transmission device and includes a first rotating member, a second rotating member, a third rotating member, a first transmission assembly, and a second transmission assembly. The first transmission assembly is connected to a first cable and a second cable and further connected to the first rotating member and the third rotating member. The second transmission assembly is connected to a third cable and a fourth cable and further connected to the second rotating member and the third rotating member. The first rotating member is rotatable to pull in one of the first cable and the second cable and to release another of the first cable and the second cable concurrently via the first transmission assembly. The second rotating member is rotatable to pull in one of the third cable and the fourth cable and to release another of the third cable and the fourth cable concurrently via the second transmission assembly. The third rotating member is rotatable to perform via the first transmission assembly and the second transmission assembly: pulling in at least one of the first cable and the second cable while releasing at least one of the third cable and the fourth cable; or pulling in at least one of the third cable and the fourth cable while releasing at least one of the first cable and the second cable.

According to an embodiment of the present disclosure, the rear-end transmission device is connected to four driving cables of the wrist mechanism, such that the wrist mechanism works cooperatively with the rear-end transmission device to achieve pitch, yaw, and grip of the wrist mechanism by pulling in or releasing the driving cables (i.e., the first cable, the second cable, the third cable, and the fourth cable), which is simple in structure and accurate in transmission. In addition, a rotation angle of the rotating member is linearly related to a pitch angle, a yaw angle, or a grip angle of the wrist mechanism, and therefore, equal-length release and/or pulling-in of the driving cables can be ensured even when the above-mentioned angles of the wrist mechanism vary in a wide range.

In some embodiments, the first rotating member is rotatable around a first central axis of the first rotating member, the second rotating member is rotatable around a second central axis of the second rotating member, and the third rotating member is rotatable around a third central axis of the third rotating member; and the first central axis is parallel to the second central axis and parallel to the third central axis.

In some embodiments, the first transmission assembly includes a first movable pulley, a first transmission cable, a second movable pulley, and a second transmission cable. The first movable pulley is connected to the first cable. The first transmission cable rides on the first movable pulley, where the first transmission cable has one end connected to the first rotating member and has another end connected to the third rotating member. The second movable pulley is rotatable around a second shaft and connected to the second cable. The second transmission cable rides on the second movable pulley, where the second transmission cable has one end connected to the first rotating member and has another end connected to the third rotating member. The first transmission cable wraps around the first rotating member in a direction opposite to a direction in which the second transmission cable wraps around the first rotating member, and the first transmission cable wraps around the third rotating member in a direction same as a direction in which the second transmission cable wraps around the third rotating member. In rotation of the first rotating member or the third rotating member, the first movable pulley is driven to move by the first transmission cable and the second movable pulley is driven to move by the second transmission cable.

In some embodiments, the first transmission assembly further includes a first guide pulley, where a portion of the first transmission cable located between the first rotating member and the first movable pulley rides on the first guide pulley, such that a portion of the first transmission cable located between the first guide pulley and the first movable pulley is parallel to a portion of the first transmission cable located between the first movable pulley and the third rotating member.

In some embodiments, a portion of the second transmission cable located between the first rotating member and the second movable pulley is parallel to a portion of the second transmission cable located between the second movable pulley and the third rotating member and parallel to the portion of the first transmission cable located between the first movable pulley and the third rotating member.

In some embodiments, the second transmission assembly includes a third movable pulley, a third transmission cable, a fourth movable pulley, and a fourth transmission cable. The third movable pulley is connected to the third cable. The third transmission cable rides on the third movable pulley, where the third transmission cable has one end connected to the second rotating member and has another end connected to the third rotating member. The fourth movable pulley is rotatable around a fourth shaft and connected to the fourth cable. The fourth transmission cable rides on the fourth movable pulley, where the fourth transmission cable has one end connected to the second rotating member and has another end connected to the third rotating member. The third transmission cable wraps around the second rotating member in a direction opposite to a direction in which the fourth transmission cable wraps around the second rotating member, and each of the third transmission cable and the fourth transmission cable wraps around the third rotating member in a direction opposite to a direction in which each of the first transmission cable and the second transmission cable wraps around the third rotating member. In rotation of the second rotating member or the third rotating member, the third movable pulley is driven to move by the third transmission cable and the fourth movable pulley is driven to move by the fourth transmission cable.

In some embodiments, the first movable pulley is pivotably connected to a first pulley seat via a first shaft, and the first pulley seat is connected to the first cable, where the second movable pulley is pivotably connected to a second pulley seat via a second shaft, and the second pulley seat is connected to the second cable, where the first shaft is parallel to the second shaft and parallel to the first central axis; and/or the third movable pulley is pivotably connected to a third pulley seat via a third shaft, and the third pulley seat is connected to the third cable, where the fourth movable pulley is pivotably connected to a fourth pulley seat via a fourth shaft, and the fourth pulley seat is connected to the fourth cable, where the third shaft is parallel to the fourth shaft and parallel to the first central axis.

In some embodiments, the second transmission assembly further includes a second guide pulley, where a portion of the fourth transmission cable located between the second rotating member and the fourth movable pulley rides on the second guide pulley, such that a portion of the fourth transmission cable located between the second guide pulley and the fourth movable pulley is parallel to a portion of the fourth transmission cable located between the fourth movable pulley and the third rotating member.

In some embodiments, a portion of the third transmission cable located between the second rotating member and the third movable pulley is parallel to a portion of the third transmission cable located between the third movable pulley and the third rotating member, and parallel to the portion of the fourth transmission cable located between the fourth movable pulley and the third rotating member.

Embodiments of the disclosure further provide a medical device including the rear-end transmission device described in any embodiment of the disclosure.

Embodiments of the disclosure further provide a surgical robot including the medical device described in any embodiment of the disclosure.

### BRIEF DESCRIPTION OF THE DRAWINGS

The following drawings of the disclosure are herein used as a part of the disclosure for understanding the disclosure. Embodiments of the present disclosure are described with reference to the accompanying drawings to explain the principles of the present disclosure, in which:
FIG. 1 is a schematic structural view of a surgical robot according to embodiments of the present disclosure;
FIG. 2 is a schematic partial structural view of the surgical robot of FIG. 1 including a wrist mechanism, a first cable, a second cable, a third cable, and a fourth cable;
FIG. 3 is another schematic partial structural view of the surgical robot of FIG. 1 including a rear-end transmission device, a first cable, a second cable, a third cable, and a fourth cable; and
FIG. 4 is a schematic partial structural view of the rear-end transmission device of the surgical robot in FIG. 1.

Reference numbers are illustrated as follows:

| | | | |
|---|---|---|---|
| 100: | Surgical robot | 110: | Robotic arm |
| 111: | Link | 120: | Medical Device |
| 130: | Wrist mechanism | 131: | Proximal clevis |
| 132: | Distal clevis | 133: | Effector |
| 134: | Upper claw | 135: | Lower claw |
| 136: | First bolt | 137: | Second bolt |
| 140: | Main shaft | 141: | First cable |
| 142: | Second cable | 143: | Third cable |
| 144: | Fourth cable | 150: | Rear-end transmission device |
| 151: | First rotating member | 152: | Second rotating member |
| 153: | Third rotating member | 156: | First movable pulley |
| 157: | Second movable pulley | 158: | First transmission cable |
| 159: | Second transmission cable | 161: | First shaft |
| 162: | Second shaft | 163: | First guide pulley |
| 164: | Third movable pulley | 165: | Fourth movable pulley |
| 166: | Third transmission cable | 167: | Fourth transmission cable |
| 168: | Third shaft | 169: | Fourth shaft |
| 171: | Second guide pulley | 172: | Chassis |
| 173: | First rotating shaft | 174: | Second rotating shaft |
| 175: | Third rotating shaft | 176: | Third guide pulley set |
| 177: | Fifth shaft | 178: | Third guide pulley |
| 181: | First pulley seat | 182: | Second pulley seat |
| 183: | Third pulley seat | 184: | Fourth pulley seat |
| A1: | First central axis | A2: | Second central axis |
| A3: | Third central axis | 180: | Base |

### DETAILED DESCRIPTION OF THE EMBODIMENTS

Detailed illustration is given in the following description to provide a more thorough understanding of the disclosure. However, it will be apparent to those skilled in the art that embodiments of the present disclosure may be practiced without one or more of these details. In other examples, some technical features well known in the art are not described in order to avoid confusion with embodiments of the present disclosure.

In order to fully understand the embodiments of the present disclosure, detailed structures will be set forth in the following description. Apparently, the implementation of the embodiments of the present disclosure is not limited to particular details familiar to those skilled in the art. It is to be noted that ordinal numbers such as "first" and "second" referenced in the disclosure are merely identifiers and do not have any other meaning, e.g., does not represent a specific order. In addition, for example, the term "first component/member" does not imply the existence of a "second component/member", and the term "second component/member" does not imply the existence of the "first component/member". The terms "up", "down", "front", "back", "left", "right", and similar expressions used in the disclosure are merely intended to explain the disclosure rather than limit the disclosure.

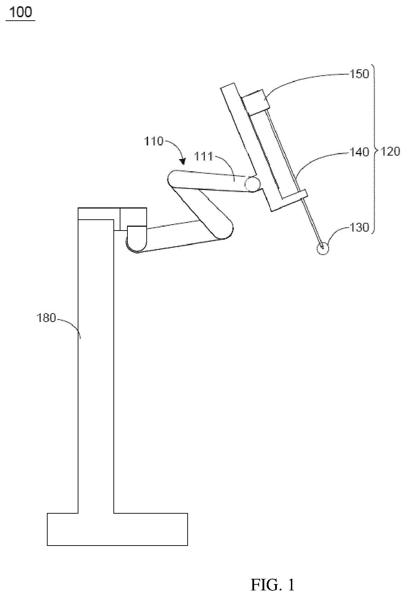

As shown in FIG. 1, embodiments of the present disclosure provide a rear-end transmission device 150, a medical device 120, and a surgical robot 100. The surgical robot 100 includes a movable robotic arm 110 and a medical device 120 mounted to the robotic arm 110. The medical device 120 includes the rear-end transmission device 150. As known in the art, the medical device 120 can achieve a number of functions, and includes but is not limited to a pair of surgical forceps or grippers of different shapes and sizes, a needle driver, a pair of scissors, or a cauterizing tool.

As shown in FIG. 1, the surgical robot 100 mainly includes a base 180, at least one robotic arm 110 rotatably disposed at an upper end of the base 180, and at least one medical device 120. Each medical device 120 is mounted to a port of a respective robotic arm 110. Merely one robotic arm 110 is exemplarily shown in FIG. 1, the robotic arm 110 includes a plurality of links 111 connected sequentially, and each of the plurality of links 111 is rotatable. In a case where the surgical robot 100 includes multiple robotic arms 110, each robotic arm 110 is provided with a respective medical device 120 which is mounted to a port of the robotic arm 110. Each medical device 120 is detachably coupled to the respective robotic arm 110, to facilitate replacement or repairment of the medical device 120. Therefore, the medical device 120 mounted to the respective robotic arm 110 may be used for a particular medical procedure or may be changed during the medical procedure to provide a desired clinical function.

The robotic arm 110 includes at least one docking port. The at least one docking port generally includes an output of a driving motor configured to provide mechanical power for operation of the medical device 120. The at least one docking port may further include an electrical interface to which the medical device 120 is coupled, to identify a type of a device coupled to the docking port and to obtain parameters of the device.

The medical device 120 generally includes the rear-end transmission device 150, a main shaft 140 extending from the rear-end transmission device 150, and a wrist mechanism 130 at a distal end of the main shaft 140. Driving cables (specifically including a first cable 141, a second cable 142, a third cable 143, and a fourth cable 144) and an electrical conductor that are connected to the wrist mechanism 130 can run through the main shaft 140 and are connected to the rear-end transmission device 150. The rear-end transmission device 150 is configured to provide mechanical coupling between the above-described driving cables and a motor driving shaft of the driving motor, so as to operate the wrist mechanism 130 by controlling movement and tension of the driving cables. The main shaft 140 is hollow and may be rigid or flexible.

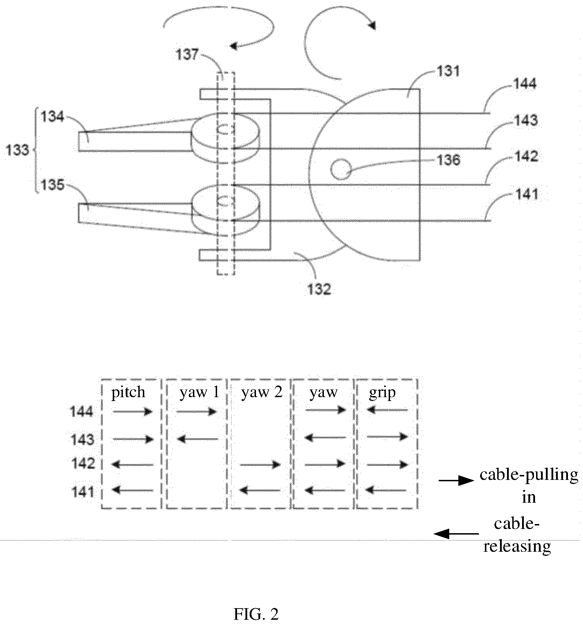

As shown in FIG. 2, the wrist mechanism 130 includes a proximal clevis 131, a distal clevis 132, and an effector 133. The distal clevis 132 is pivotably connected to the proximal clevis131 via a first bolt 136, such that pitch of the wrist mechanism 130 is realized through rotation of the distal clevis 132 relative to the proximal clevis 131. The effector 133 includes an upper claw 134 and a lower claw 135. Each of the upper claw 134 and the lower claw 135 is pivotably connected to the distal clevis 132 via a second bolt 137, such that grip and yaw of the wrist mechanism 130 are realized through rotation of the claws 134, 135 relative to the distal clevis 132. In some embodiments, the second bolt 137 is perpendicular to the first bolt136.

The first cable 141 and the second cable 142 are wound on and connected to the lower claw 135 of the effector 133. That is, the first cable 141 and the second cable 142 may be formed integrally from one continuous cable wrapped around the lower claw 135. The third cable 143 and the fourth cable 144 are wound on and connected to the upper claw 134 of the effector 133. That is, the third cable 143 and the fourth cable 144 may be formed integrally from one continuous cable wrapped around the upper claw 134. The first cable 141, the second cable 142, the third cable 143, and the fourth cable 144 are extended along hard surfaces of guide passages (not shown) defined by the effector 133, the distal clevis 132, and the proximal clevis 131, and then reach the rear-end transmission device 150 along the main shaft 140. The guide passage may be a groove having a U-shaped or semicircular cross section.

Continuing with reference to FIG. 2, by pulling in the third cable 143 and the fourth cable 144 in equal lengths while releasing the first cable 141 and the second cable 142 in equal lengths, the distal clevis 132 is rotated clockwise around the first bolt 136 relative to the proximal clevis131, to achieve pitch of the wrist mechanism 130 in a direction (referring to FIG. 2). Similarly, by pulling in the first cable 141 and the second cable 142 in equal lengths while releasing the third cable 143 and the fourth cable 144 in equal lengths, the distal clevis 132 is rotated counterclockwise around the first bolt 136 relative to the proximal clevis 131, to achieve pitch of the wrist mechanism 130 in a reverse direction.

The upper claw 134 is rotated clockwise around the second bolt 137 relative to the distal clevis 132 by pulling in the fourth cable 144 and releasing the third cable 143 concurrently in equal lengths (referring to yaw 1 in FIG. 2). Similarly, the upper claw 134 is rotated counterclockwise around the second bolt 137 relative to the distal clevis 132 by pulling in the third cable 143 and releasing the fourth cable 144 concurrently in equal lengths. The lower claw 135 is rotated clockwise around the second bolt 137 relative to the distal clevis 132 by pulling in the second cable 142 and releasing the first cable 141 concurrently in equal lengths (referring to yaw 2 in FIG. 2). Similarly, the lower claw 135 is rotated counterclockwise around the second bolt 137 relative to the distal clevis 132 by pulling in the first cable 141 and releasing the second cable 142 concurrently in equal lengths. The effector 133 can achieve yaw and grip through aggregate motion of the upper claw 134 and the lower claw 135, which is described in detail below.

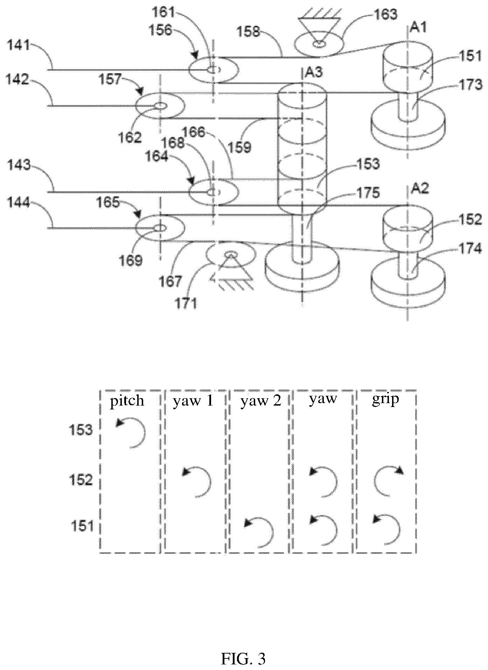

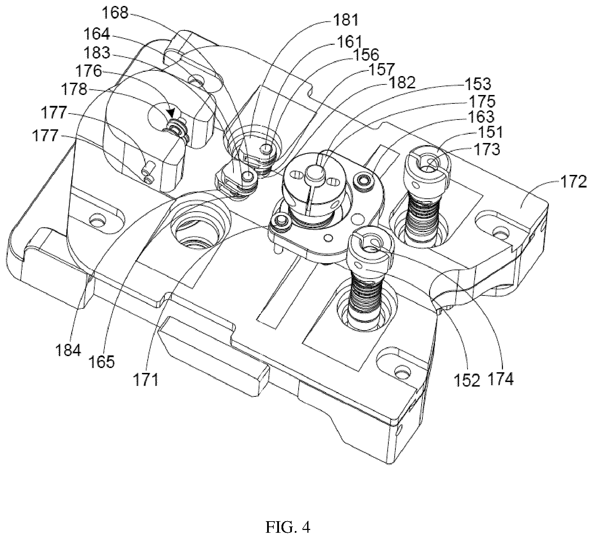

As shown in FIGS. 3 and 4, the rear-end transmission device 150 mainly includes a first rotating member 151, a second rotating member 152, a third rotating member 153, a first transmission assembly, a second transmission assembly, a first rotating shaft 173, a second rotating shaft 174, a third rotating shaft 175, and a chassis 172. Each of the first rotating shaft 173, the second rotating shaft 174, and the third rotating shaft 175 is rotatably mounted to the chassis 172, and connected with a respective driving motor. The first transmission assembly and the second transmission assembly are each mounted to the chassis 172. The chassis 172 has a plurality of interfaces and each of the plurality of interfaces corresponds to one driving motor, such that each of the first rotating shaft 173, the second rotating shaft 174, and the third rotating shaft 175 can be stably connected with the corresponding driving motor when the medical device 120 is properly mounted, so as to realize the transmission of rotational motion.

The surgical robot 100 may include at least three driving motors. The three driving motors are connected to the first rotating shaft 173, the second rotating shaft 174, and the third rotating shaft 175, respectively. The first rotating shaft 173, the second rotating shaft 174, and the third rotating shaft 175 are arranged parallel to each other. The first rotating shaft 173, the second rotating shaft 174, and the third rotating shaft 175 are arranged at three tips of a substantial triangle, respectively. It shall be understood that there is no restriction on arrangement of the first rotating shaft 173, the second rotating shaft 174, and the third rotating shaft 175. The first rotating shaft 173, the second rotating shaft 174, and the third rotating shaft 175 may also be arranged in a straight line as required. The first rotating shaft 173 is fixedly connected to the first rotating member 151 to drive rotation of the first rotating member 151. The second rotating shaft 174 is fixedly connected to the second rotating member 152 to drive rotation of the second rotating member 152. The third rotating shaft 175 is fixedly connected to the third rotating member 153, to drive rotation of the third rotating member 153. In embodiments of the disclosure, each of the first rotating member 151, the second rotating member 152, and the third rotating member 153 is a winch.

The first transmission assembly is connected to the first cable 141 and the second cable 142 and further connected to the first rotating member 151 and the third rotating member 153. The first transmission assembly mainly includes a first movable pulley 156, a second movable pulley 157, a first transmission cable 158, and a second transmission cable 159. The first movable pulley 156 is pivotably connected to the first pulley seat 181 via the first shaft 161, the first movable pulley 156 is pivotable around the first shaft 161, and the first cable 141 is connected to the first pulley seat 181. The second movable pulley 157 is pivotably connected to the second pulley seat 182 via the second shaft 162, the second movable pulley 157 is pivotable around the second shaft 162, and the second cable 142 is connected to the second pulley seat 182.

The first transmission cable 158 rides on the first movable pulley 156. The first transmission cable 158 has one end connected to the first rotating member 151 and has the other end connected to the third rotating member 153. The second transmission cable 159 rides on the second movable pulley 157. The second transmission cable 159 has one end connected to the first rotating member 151 and has the other end connected to the third rotating member 153. Specifically, the first transmission cable 158 and the second transmission cable 159 wrap around the first rotating member 151 in opposite directions and wrap around the third rotating member 153 in a same direction. In rotation of the first rotating member 151 or the third rotating member 153, the first movable pulley 156 is driven to move by the first transmission cable 158, and the second movable pulley 157 is driven to move by the second transmission cable 159.

The first transmission assembly further includes a first guide pulley 163. A portion of the first transmission cable 158 located between the first rotating member 151 and the first movable pulley 156 rides on the first guide pulley 163. Therefore, a portion of the first transmission cable 158 located between the first guide pulley 163 and the first movable pulley 156 is parallel to another portion of the first transmission cable 158 located between the first movable pulley 156 and the third rotating member 153, and an absolute value of a linear speed of the portion of the first transmission cable 158 located between the first guide pulley 163 and the first movable pulley 156 is equal to an absolute value of a linear speed of the another portion of the first transmission cable 158 located between the first movable pulley 156 and the third rotating member 153. In other words, the linear speed of the portion of the first transmission cable 158 on one side of two opposite sides of the first movable pulley 156 and the linear speed of the another portion of the first transmission cable 158 on the other side of the two opposite sides of the first movable pulley 156 have a same absolute value. A portion of the second transmission cable 159 located between the first rotating member 151 and the second movable pulley 157 is parallel to another portion of the second transmission cable 159 located between the second movable pulley 157 and the third rotating member 153, and parallel to the another portion of the first transmission cable 158 located between the first movable pulley 156 and the third rotating member 153. In other words, a linear speed of the portion of the second transmission cable 159 located on one side of two opposite sides of the second movable pulley 157 and a linear speed of the another portion of the second transmission cable 159 located on the other side of the two opposite sides of the second movable pulley 157 have a same absolute value. The first guide pulley 163 may be a fixed pulley. It shall be understood that additional guide pulleys may be employed as needed to guide the first transmission cable 158 and the second transmission cable 159.

The first rotating member 151 is rotatable around a first central axis A1 of the first rotating member 151, to pull in one of the first cable 141 and the second cable 142 while releasing the other of the first cable 141 and the second cable 142 through the first transmission assembly, so as to achieve clockwise or counterclockwise rotation of the lower claw 135 around the second bolt 137 relative to the distal clevis 132. In an embodiment, the first shaft 161 is parallel to the second shaft 162 and parallel to the first central axis A1.

Specifically, during stationary of the third rotating member 153, when the driving motor controls the first rotating shaft 173 to drive the first rotating member 151 to rotate counterclockwise, the second transmission cable 159 is pulled in (i.e., wrapped around/wound on the first rotating member 151) and the first transmission cable 158 is released in equal lengths concurrently. In this way, the second cable 142 is pulled in via the second movable pulley 157 and the first cable 141 is released in equal lengths concurrently via the first movable pulley 156, thereby realizing clockwise rotation of the lower claw 135 around the second bolt 137 relative to the distal clevis 132 (referring to yaw 2 in FIG. 2). During stationary of the third rotating member 153, when the driving motor controls the first rotating shaft 173 to drive the first rotating member 151 to rotate clockwise, the first transmission cable 158 is pulled in (i.e., wrapped around/wound on the first rotating member 151) and the second transmission cable 159 is released in equal lengths concurrently. In this way, the first cable 141 is pulled in via the first movable pulley 156 and the second cable 142 is released in equal lengths via the second movable pulley 157 concurrently, thereby realizing counterclockwise rotation of the lower claw 135 around the second bolt 137 relative to the distal clevis 132.

With continued reference to FIGS. 3 and 4, the second transmission assembly is connected to the third cable 143 and the fourth cable 144, and further connected to the second rotating member 152 and the third rotating member 153. The second transmission assembly includes a third movable pulley 164, a fourth movable pulley 165, a third transmission cable 166, and a fourth transmission cable 167. The third movable pulley 164 is pivotably connected to a third pulley seat 183 via a third shaft 168, the third movable pulley 164 is pivotable around the third shaft 168, and the third cable 143 is connected to the third pulley seat 183. The fourth movable pulley 165 is pivotably connected to a fourth pulley seat 184 via a fourth shaft 169, the fourth movable pulley 165 is pivotable around the fourth shaft 169, and the fourth cable 144 is connected to the fourth pulley seat 184.

The third transmission cable 166 rides on the third movable pulley 164. The third transmission cable 166 has one end connected to the second rotating member 152 and has the other end connected to the third rotating member 153. The fourth transmission cable 167 rides on the fourth movable pulley 165. The fourth transmission cable 167 has one end connected to the second rotating member 152 and has the other end connected to the third rotating member 153. Specifically, the third transmission cable 166 and the fourth transmission cable 167 wraps around the second rotating member 152 in opposite directions, and a direction in which the third transmission cable 166 and the fourth transmission cable 167 wrap around the third rotating member 153 is opposite to a direction in which the first transmission cable 158 and the second transmission cable 159 wrap around the third rotating member 153. In other words, the third transmission cable 166 and the fourth transmission cable 167 wrap around the third rotating member 153 in a same direction. In rotation of the second rotating member 152 or the third rotating member 153, the third movable pulley 164 is driven to move by the third transmission cable 166, and the fourth movable pulley 165 is driven to move by the fourth transmission cable 167.

The second transmission assembly further includes a second guide pulley 171. A portion of the fourth transmission cable 167 located between the second rotating member 152 and the fourth movable pulley 165 rides on the second guide pulley 171. Therefore, a portion of the fourth transmission cable 167 located between the second guide pulley 171 and the fourth movable pulley 165 is parallel to another portion of the fourth transmission cable 167 located between the fourth movable pulley 165 and the third rotating member 153. A portion of the third transmission cable 166 located between the second rotating member 152 and the third movable pulley 164 is parallel to another portion of the third transmission cable 166 located between the third movable pulley 164 and the third rotating member 153, and parallel to the another portion of the fourth transmission cable 167 located between the fourth movable pulley 165 and the third rotating member 153. The second guide pulley 171 may be a fixed pulley. It shall be understood that additional guide pulleys may be employed as needed to guide the third transmission cable 166 and the fourth transmission cable 167.

The second rotating member 152 is rotatable around a second central axis A2 of the second rotating member 152, to pull in one of the third cable 143 and the fourth cable 144 while releasing the other of the third cable 143 and the fourth cable 144 through the second transmission assembly, so as to achieve clockwise or counterclockwise rotation of the upper claw 134 around the second bolt 137 relative to the distal clevis 132. In an embodiment, the third shaft 168 is parallel to the fourth shaft 169 and parallel to the second central axis A2.

Specifically, during stationary of the third rotating member 153, when the driving motor controls the second rotating shaft 174 to drive the second rotating member 152 to rotate counterclockwise, the fourth transmission cable 167 is pulled in (i.e., wrapped around/wound on the second rotating member 152) and the third transmission cable 166 is released in equal lengths concurrently. In this way. the fourth cable 144 is pulled in via the fourth movable pulley 165 and the third cable 143 is released in equal lengths via the third movable pulley 164 concurrently, so that the upper claw 134 is rotated clockwise around the second bolt 137 relative to the distal clevis 132 (referring to yaw 1 in FIG. 2). During stationary of the third rotating member 153, when the driving motor controls the second rotating shaft 174 to drive the second rotating member 152 to rotate clockwise, the third transmission cable 166 is pulled in (i.e., wrapped around/wound on the second rotating member 152) and the fourth transmission cable 167 is released in equal lengths concurrently. In this way, the third cable 143 is pulled in via the third movable pulley 164 and the fourth cable 144 is released in equal lengths via the fourth movable pulley 165 concurrently, so that the upper claw 134 is rotated counterclockwise around the second bolt 137 relative to the distal clevis 132.

The rear-end transmission device 150 further includes a third guide pulley set 176 (referring to FIG. 4). The third guide pulley set 176 includes two fifth shafts 177 parallel to each other, and each of the two fifth shafts 177 is provided with two third guide pulleys 178 pivotably disposed thereon. Each third guide pulley 178 may be a fixed pulley.

The four third guide pulleys 178 guide the first cable 141, the second cable 142, the third cable 143, and the fourth cable 144, respectively. Therefore, a portion of the first cable 141 located between the third guide pulley set 176 and the first movable pulley 156 is parallel to the another portion of the first transmission cable 158 located between the first movable pulley 156 and the third rotating member 153. In addition, a portion of the second cable 142 located between the third guide pulley set 176 and the second movable pulley 157 is parallel to the another portion of the second transmission cable 159 located between the second movable pulley 157 and the third rotating member 153. Furthermore, a portion of the third cable 143 located between the third guide pulley set 176 and the third movable pulley 164 is parallel to the another portion of the third transmission cable 166 located between the third movable pulley 164 and the third rotating member 153. Furthermore, a portion of the fourth cable 144 located between the third guide pulley set 176 and the fourth movable pulley 165 is parallel to the another portion of the fourth transmission cable 167 located between the fourth movable pulley 165 and the third rotating member 153. In this way, it is possible to achieve linear combination of driven of the first cable 141 and the second cable 142 by the first rotating member 151 and the third rotating member 153, and linear combination of driven of the third cable 143 and the fourth cable 144 by the second rotating member 152 and the third rotating member 153.

In this embodiment, by controlling rotation of the first rotating shaft 173 and the second rotating shaft 174, yaw and grip of the effector 133 can be realized.

Specifically, during stationary of the third rotating shaft 175, when both the first rotating shaft 173 and the second rotating shaft 174 rotate counterclockwise at equal angles, that is, when both the first rotating member 151 and the second rotating member 152 rotate counterclockwise at equal angles, both the upper claw 134 and the lower claw 135 rotate clockwise, thereby realizing yaw of the effector 133 in a direction (referring to FIG. 2). During stationary of the third rotating shaft 175, when both the first rotating shaft 173 and the second rotating shaft 174 rotate clockwise at equal angles, that is, when both the first rotating member 151 and the second rotating member 152 rotate clockwise at equal angles, both the upper claw 134 and the lower claw 135 rotate counterclockwise, thereby realizing yaw of the effector 133 in a reverse direction.

During stationary of the third rotating shaft 175, when the first rotating shaft 173 rotates counterclockwise and the second rotating shaft 174 rotates clockwise at equal angles concurrently, that is, when the first rotating member 151 rotates counterclockwise and the second rotating member 152 rotates clockwise at equal angles concurrently, the lower claw 135 rotates clockwise and the upper claw 134 rotates counterclockwise concurrently, to realize grip of the effector 133 (referring to FIG. 2). During stationary of the third rotating shaft 175, when the first rotating shaft 173 rotates clockwise and the second rotating shaft 174 rotates counterclockwise at equal angles concurrently, that is, when the first rotating member 151 rotates clockwise and the second rotating member 152 rotates counterclockwise at equal angles concurrently, the lower claw 135 rotates counterclockwise and the upper claw 134 rotates clockwise, to realize releasing of the grip of the effector 133.

The third rotating member 153 is rotatable around a third central axis A3 of the third rotating member 153 to perform via the first transmission assembly and the second transmission assembly: pulling in at least one of the first cable 141 and the second cable 142 while releasing at least one of the third cable 143 and the fourth cable 144; or pulling in at least one of the third cable 143 and the fourth cable 144 while releasing at least one of the first cable 141 and the second cable 142, to achieve pitch of the wrist mechanism 130. In an embodiment, the first central axis A1 is parallel to the second central axis A2 and parallel to the third central axis A3.

In the present embodiment, during rotation of the third rotating member 153, the first cable 141 and the second cable 142 can be pulled in in equal lengths and the third cable 143 and the fourth cable 144 can be released in equal lengths concurrently via the first transmission assembly and the second transmission assembly, and during the rotation of the third rotating member 153, a total length of a portion of the first cable 141 and a portion of the second cable 142 that are pulled in is equal to a total length of a portion of the third cable 143 and a portion of the fourth cable 144 that are released, thereby achieving pitch of the wrist mechanism 130. Alternatively, during rotation of the third rotating member 153, the third cable 143 and the fourth cable 144 can be pulled in in equal lengths and the first cable 141 and the second cable 142 can be released in equal lengths concurrently, and during rotation of the third rotating member 153, a total length of a portion of the third cable 143 and a portion of the fourth cable 144 that are pulled in is equal to a total length of a portion of the first cable 141 and a portion of the second cable 142 that are released, thereby achieving pitch of the wrist mechanism 130.

Specifically, when the first rotating shaft 173 and the second rotating shaft 174 remain stationary and the driving motor controls the third rotating shaft 175 to drive the third rotating member 153 to rotate counterclockwise, the first transmission cable 158 and the second transmission cable 159 are released in equal lengths, and the third transmission cable 166 and the fourth transmission cable 167 are pulled in in equal lengths concurrently. That is, the first cable 141 and the second cable 142 are released in equal lengths and the third cable 143 and the fourth cable 144 are pulled in in equal lengths concurrently, thereby realizing the pitch of the wrist mechanism 130 in a direction. Similarly, when the first rotating shaft 173 and the second rotating shaft 174 remain stationary and the driving motor controls the third rotating shaft 175 to drive the third rotating member 153 to rotate clockwise, the first transmission cable 158 and the second transmission cable 159 are pulled in in equal lengths and the third transmission cable 166 and the fourth transmission cable 167 are released in equal lengths concurrently. That is, the first cable 141 and the second cable 142 are pulled in in equal lengths and the third cable 143 and the fourth cable 144 are released in equal lengths concurrently, thereby realizing the pitch of the wrist mechanism 130 in a reverse direction.

In the present embodiment, since rotation of the first rotating member 151, the second rotating member 152, and the third rotating member 153 are transmitted to the first cable 141, the second cable 142, the third cable 143, and the fourth cable 144 via the first movable pulley 156, the second movable pulley 157, the third movable pulley 164, and the fourth movable pulley 165, moving lengths (pulled-in or released lengths) of the first cable 141, the second cable 142, the third cable 143, and the fourth cable 144 are respectively half of moving lengths of the first transmission cable 158, the second transmission cable 159, the third transmission cable 166, and the fourth transmission cable 167, which means that a ratio of an input speed of the rear-end transmission device to an output speed of the rear-end transmission device is 2: 1. Therefore, when there is a return difference in a reducer of the driving motor, or when there is a return difference at a connection between at least one of the first rotating shaft 173, the second rotating shaft 174, and the third rotating shaft 175 and the respective driving motors, the influence of the return difference on the wrist mechanism 130 at the front end is reduced by half, so that the motion accuracy of the wrist mechanism 130 may be improved.

According to an embodiment of the present disclosure, the rear-end transmission device is connected to four driving cables of the wrist mechanism, such that the wrist mechanism works cooperatively with the rear-end transmission device to achieve pitch, yaw, and grip of the wrist mechanism by pulling in or releasing the driving cables (i.e., the first cable, the second cable, the third cable, and the fourth cable) in equal lengths, which is simple in structure and accurate in transmission. In addition, a rotation angle of the rotating member is linearly related to a pitch angle, a yaw angle, or a grip angle of the wrist mechanism, and therefore, equal-length release and/or pulling-in of the driving cables can be ensured even when the above-mentioned angles of the wrist mechanism vary in a wide range.

Unless otherwise defined, technical and scientific terms used herein have the same meanings as are commonly understood by those skilled in the art of the present disclosure. Terms used herein are for specific practical purposes only and are not intended to limit the present disclosure. Terms such as "disposed/disposing" that appear herein may denote either a part is attached directly to another part or a part is attached to another part through middleware. Features described herein in an embodiment may be applied to another embodiment alone or in combination with other features unless the features are not applicable in the other embodiment or otherwise noted.

The present disclosure has been described by way of the above-described embodiments but it shall be understood that the above-described embodiments are for the purpose of illustrative and illustration only and are not intended to limit the present disclosure to the scope of the described embodiments. Those skilled in the art will appreciate that many more variations and modifications may be made according to the teachings of the present disclosure, all of which fall within the scope of the claims of the present disclosure.

## Claims

1. A rear-end transmission device (150), comprising:
a first rotating member (151);
a second rotating member (152);
a third rotating member (153);
a first transmission assembly connected to a first cable (141) and a second cable (142) and further connected to the first rotating member (151) and the third rotating member (153); and
a second transmission assembly connected to a third cable (143) and a fourth cable (144) and further connected to the second rotating member (152) and the third rotating member (153);
wherein the first rotating member (151) is rotatable to pull in one of the first cable (141) and the second cable (142) and to release another of the first cable (141) and the second cable (142) concurrently via the first transmission assembly;
the second rotating member (152) is rotatable to pull in one of the third cable (143) and the fourth cable (144) and to release another of the third cable (143) and the fourth cable (144) concurrently via the second transmission assembly; and
the third rotating member (153) is rotatable to perform via the first transmission assembly and the second transmission assembly:
pulling in the first cable (141) and the second cable (142) while releasing the third cable (143) and the fourth cable (144); or
pulling in the third cable (143) and the fourth cable (144) while releasing the first cable (141) and the second cable (142);
**characterized in that**, the first transmission assembly comprises:
a first movable pulley (156) connected to the first cable (141);
a first transmission cable (158) riding on the first movable pulley (156), wherein the first transmission cable (158) has one end connected to the first rotating member (151) and has another end connected to the third rotating member (153);
a second movable pulley (157) connected to the second cable (142); and
a second transmission cable (159) riding on the second movable pulley (157), wherein the second transmission cable (159) has one end connected to the first rotating member (151) and has another end connected to the third rotating member (153); wherein
the first transmission cable (158) wraps around the first rotating member (151) in a direction opposite to a direction in which the second transmission cable (159) wraps around the first rotating member (151), and the first transmission cable (158) wraps around the third rotating member (153) in a direction same as a direction in which the second transmission cable (159) wraps around the third rotating member (153); and
in rotation of the first rotating member (151) or the third rotating member (153), the first movable pulley (156) is driven to move by the first transmission cable (158) and the second movable pulley (157) is driven to move by the second transmission cable (159);
the second transmission assembly comprises:
a third movable pulley (164) connected to the third cable (143);
a third transmission cable (166) riding on the third movable pulley (164), wherein the third transmission cable (166) has one end connected to the second rotating member (152) and has another end connected to the third rotating member (153);
a fourth movable pulley (165) connected to the fourth cable (144); and
a fourth transmission cable (167) riding on the fourth movable pulley (165), wherein the fourth transmission cable (167) has one end connected to the second rotating member (152) and has another end connected to the third rotating member (153); wherein
the third transmission cable (166) wraps around the second rotating member (152) in a direction opposite to a direction in which the fourth transmission cable (167) wraps around the second rotating member (152), and each of the third transmission cable (166) and the fourth transmission cable (167) wraps around the third rotating member (153) in a direction opposite to a direction in which each of the first transmission cable (158) and the second transmission cable (159) wraps around the third rotating member (153); and
in rotation of the second rotating member (152) or the third rotating member (153), the third movable pulley (164) is driven to move by the third transmission cable (166) and the fourth movable pulley (165) is driven to move by the fourth transmission cable (167).

2. The rear-end transmission device (150) of claim 1, wherein the first rotating member (151) is rotatable around a first central axis (A1) of the first rotating member (151), the second rotating member (152) is rotatable around a second central axis (A2) of the second rotating member (152), and the third rotating member (153) is rotatable around a third central axis (A3) of the third rotating member (153); and
wherein the first central axis (A1) is parallel to the second central axis (A2) and parallel to the third central axis (A3).

3. The rear-end transmission device (150) of claim 1, wherein the first transmission assembly further comprises a first guide pulley (163), and wherein
a portion of the first transmission cable (158) located between the first rotating member (151) and the first movable pulley (156) rides on the first guide pulley (163), such that a portion of the first transmission cable (158) located between the first guide pulley (163) and the first movable pulley (156) is parallel to a portion of the first transmission cable (158) located between the first movable pulley (156) and the third rotating member (153).

4. The rear-end transmission device (150) of claim 3, wherein a portion of the second transmission cable (159) located between the first rotating member (151) and the second movable pulley (157) is parallel to a portion of the second transmission cable (159) located between the second movable pulley (157) and the third rotating member (153) and parallel to the portion of the first transmission cable (158) located between the first movable pulley (156) and the third rotating member (153).

5. The rear-end transmission device (150) of claim 1, wherein
the first movable pulley (156) is pivotably connected to a first pulley seat (181) via a first shaft (161), and the first pulley seat (181) is connected to the first cable (141), and wherein the second movable pulley (157) is pivotably connected to a second pulley seat (182) via a second shaft (162), and the second pulley seat (182) is connected to the second cable (142), wherein the first shaft (161) is parallel to the second shaft (162) and parallel to the first central axis (A1).

6. The rear-end transmission device (150) of claim 5, wherein the third movable pulley (164) is pivotably connected to a third pulley seat (183) via a third shaft (168), and the third pulley seat (183) is connected to the third cable (143), and wherein the fourth movable pulley (165) is pivotably connected to a fourth pulley seat (184) via a fourth shaft (169), and the fourth pulley seat (184) is connected to the fourth cable (144), wherein the third shaft (168) is parallel to the fourth shaft (169) and parallel to the first central axis (A1).

7. The rear-end transmission device (150) of any of claims 5 to 6, wherein the second transmission assembly further includes a second guide pulley (171), and wherein
a portion of the fourth transmission cable (167) located between the second rotating member (152) and the fourth movable pulley (165) rides on the second guide pulley (171), such that a portion of the fourth transmission cable (167) located between the second guide pulley (171) and the fourth movable pulley (165) is parallel to a portion of the fourth transmission cable (167) located between the fourth movable pulley (165) and the third rotating member (153).

8. The rear-end transmission device (150) of claim 7, wherein a portion of the third transmission cable (166) located between the second rotating member (152) and the third movable pulley (164) is parallel to a portion of the third transmission cable (166) located between the third movable pulley (164) and the third rotating member (153), and parallel to the portion of the fourth transmission cable (167) located between the fourth movable pulley (165) and the third rotating member (153).

9. A medical device (120), comprising the rear-end transmission device (150) of any of claims 1 to 8.

10. A surgical robot (100), comprising the medical device (120) of claim 9.

## Patentansprüche

1. Hinterende-Übertragungsvorrichtung (150), umfassend:
ein erstes Drehelement (151);
ein zweites Drehelement (152);
ein drittes Drehelement (153);
eine erste Übertragungsbaugruppe, die mit einem ersten Kabel (141) und einem zweiten Kabel (142) verbunden ist und ferner mit dem ersten Drehelement (151) und dem dritten Drehelement (153) verbunden ist; und
eine zweite Übertragungsbaugruppe, die mit einem dritten Kabel (143) und einem vierten Kabel (144) verbunden ist und ferner mit dem zweiten Drehelement (152) und dem dritten Drehelement (153) verbunden ist;
wobei das erste Drehelement (151) dazu drehbar ist, eines von dem ersten Kabel (141) und dem zweiten Kabel (142) einzuziehen und gleichzeitig über die erste Übertragungsbaugruppe ein anderes von dem ersten Kabel (141) und dem zweiten Kabel (142) freizugeben;
das zweite Drehelement (152) dazu drehbar ist, eines von dem dritten Kabel (143) und dem vierten Kabel (144) einzuziehen und gleichzeitig über die zweite Übertragungsbaugruppe ein anderes von dem dritten Kabel (143) und dem vierten Kabel (144) freizugeben; und
das dritte Drehelement (153) dazu drehbar ist, über die erste Übertragungsbaugruppe und die zweite Übertragungsbaugruppe Folgendes durchzuführen:
Einziehen des ersten Kabels (141) und des zweiten Kabels (142), während des Freigebens des dritten Kabels (143) und des vierten Kabels (144); oder
Einziehen des dritten Kabels (143) und des vierten Kabels (144), während des Freigebens des ersten Kabels (141) und des zweiten Kabels (142);
**gekennzeichnet dadurch, dass** die erste Übertragungsbaugruppe Folgendes umfasst:
eine erste bewegliche Rolle (156), die mit dem ersten Kabel (141) verbunden ist;
ein erstes Übertragungskabel (158), das auf der ersten beweglichen Rolle (156) läuft, wobei das erste Übertragungskabel (158) ein Ende aufweist, das mit dem ersten Drehelement (151) verbunden ist, und ein anderes Ende aufweist, das mit dem dritten Drehelement (153) verbunden ist;
eine zweite bewegliche Rolle (157), die mit dem zweiten Kabel (142) verbunden ist; und
ein zweites Übertragungskabel (159), das auf der zweiten beweglichen Rolle (157) läuft, wobei das zweite Übertragungskabel (159) ein Ende aufweist, das mit dem ersten Drehelement (151) verbunden ist, und ein anderes Ende aufweist, das mit dem dritten Drehelement (153) verbunden ist; wobei
das erste Übertragungskabel (158) sich um das erste Drehelement (151) in einer Richtung wickelt, die einer Richtung entgegengesetzt ist, in der sich das zweite Übertragungskabel (159) um das erste Drehelement (151) wickelt, und das erste Übertragungskabel (158) sich um das dritte Drehelement (153) in einer Richtung wickelt, die dieselbe Richtung ist, in der sich das zweite Übertragungskabel (159) um das dritte Drehelement (153) wickelt; und
bei Drehung des ersten Drehelements (151) oder des dritten Drehelements (153) die erste bewegliche Rolle (156) von dem ersten Übertragungskabel (158) dazu angetrieben wird, sich zu bewegen, und die zweite bewegliche Rolle (157) von dem zweiten Übertragungskabel (159) dazu angetrieben wird, sich zu bewegen;
die zweite Übertragungsbaugruppe Folgendes umfasst:
eine dritte bewegliche Rolle (164), die mit dem dritten Kabel (143) verbunden ist;
ein drittes Übertragungskabel (166), das auf der dritten beweglichen Rolle (164) läuft, wobei das dritte Übertragungskabel (166) ein Ende aufweist, das mit dem zweiten Drehelement (152) verbunden ist, und ein anderes Ende aufweist, das mit dem dritten Drehelement (153) verbunden ist;
eine vierte bewegliche Rolle (165), die mit dem vierten Kabel (144) verbunden ist; und
ein viertes Übertragungskabel (167), das auf der vierten beweglichen Rolle (165) läuft, wobei das vierte Übertragungskabel (167) ein Ende aufweist, das mit dem zweiten Drehelement (152) verbunden ist, und ein anderes Ende aufweist, das mit dem dritten Drehelement (153) verbunden ist; wobei
das dritte Übertragungskabel (166) sich um das zweite Drehelement (152) in einer Richtung wickelt, die einer Richtung entgegengesetzt ist, in der sich das vierte Übertragungskabel (167) um das zweite Drehelement (152) wickelt, und sich jedes von dem dritten Übertragungskabel (166) und dem vierten Übertragungskabel (167) um das dritte Drehelement (153) in einer Richtung wickelt, die entgegengesetzt zu einer Richtung ist, in der sich jedes von dem ersten Übertragungskabel (158) und dem zweiten Übertragungskabel (159) um das dritte Drehelement (153) wickelt; und
bei Drehung des zweiten Drehelements (152) oder des dritten Drehelements (153) die dritte bewegliche Rolle (164) von dem dritten Übertragungskabel (166) dazu angetrieben wird, sich zu bewegen, und die vierte bewegliche Rolle (165) von dem vierten Übertragungskabel (167) dazu angetrieben wird, sich zu bewegen.

2. Hinterende-Übertragungsvorrichtung (150) nach Anspruch 1, wobei das erste Drehelement (151) um eine erste Mittelachse (A1) des ersten Drehelements (151) drehbar ist, das zweite Drehelement (152) um eine zweite Mittelachse (A2) des zweiten Drehelements (152) drehbar ist und das dritte Drehelement (153) um eine dritte Mittelachse (A3) des dritten Drehelements (153) drehbar ist; und
wobei die erste Mittelachse (A1) parallel zu der zweiten Mittelachse (A2) und parallel zu der dritten Mittelachse (A3) ist.

3. Hinterende-Übertragungsvorrichtung (150) nach Anspruch 1, wobei die erste Übertragungsbaugruppe ferner eine erste Umlenkrolle (163) umfasst, und wobei
ein Abschnitt des ersten Übertragungskabels (158), der zwischen dem ersten Drehelement (151) und der ersten beweglichen Rolle (156) angeordnet ist, auf der ersten Umlenkrolle (163) läuft, sodass ein Abschnitt des ersten Übertragungskabels (158), der zwischen der ersten Umlenkrolle (163) und der ersten beweglichen Rolle (156) angeordnet ist, parallel zu einem Abschnitt des ersten Übertragungskabels (158) ist, der zwischen der ersten beweglichen Rolle (156) und dem dritten Drehelement (153) angeordnet ist.

4. Hinterende-Übertragungsvorrichtung (150) nach Anspruch 3, wobei ein Abschnitt des zweiten Übertragungskabels (159), der zwischen dem ersten Drehelement (151) und der zweiten beweglichen Rolle (157) angeordnet ist, parallel zu einem Abschnitt des zweiten Übertragungskabels (159) ist, der zwischen der zweiten beweglichen Rolle (157) und dem dritten Drehelement (153) angeordnet ist, und parallel zu dem Abschnitt des ersten Übertragungskabels (158) ist, der zwischen der ersten beweglichen Rolle (156) und dem dritten Drehelement (153) angeordnet ist.

5. Hinterende-Übertragungsvorrichtung (150) nach Anspruch 1, wobei
die erste bewegliche Rolle (156) über eine erste Welle (161) schwenkbar mit einem ersten Rollensitz (181) verbunden ist, und der erste Rollensitz (181) mit dem ersten Kabel (141) verbunden ist, und wobei die zweite bewegliche Rolle (157) über eine zweite Welle (162) schwenkbar mit einem zweiten Rollensitz (182) verbunden ist, und der zweite Rollensitz (182) mit dem zweiten Kabel (142) verbunden ist, wobei die erste Welle (161) parallel zu der zweiten Welle (162) und parallel zu der ersten Mittelachse (A1) ist.

6. Hinterende-Übertragungsvorrichtung (150) nach Anspruch 5, wobei die dritte bewegliche Rolle (164) über eine dritte Welle (168) schwenkbar mit einem dritten Rollensitz (183) verbunden ist, und der dritte Rollensitz (183) mit dem dritten Kabel (143) verbunden ist, und wobei die vierte bewegliche Rolle (165) über eine vierte Welle (169) schwenkbar mit einem vierten Rollensitz (184) verbunden ist, und der vierte Rollensitz (184) mit dem vierten Kabel (144) verbunden ist, wobei die dritte Welle (168) parallel zu der vierten Welle (169) und parallel zu der ersten Mittelachse (A1) ist.

7. Hinterende-Übertragungsvorrichtung (150) nach einem der Ansprüche 5 bis 6, wobei die zweite Übertragungsbaugruppe ferner eine zweite Umlenkrolle (171) umfasst, und wobei
ein Abschnitt des vierten Übertragungskabels (167), der zwischen dem zweiten Drehelement (152) und der vierten beweglichen Rolle (165) angeordnet ist, auf der zweiten Umlenkrolle (171) läuft, sodass ein Abschnitt des vierten Übertragungskabels (167), der zwischen der zweiten Umlenkrolle (171) und der vierten beweglichen Rolle (165) angeordnet ist, parallel zu einem Abschnitt des vierten Übertragungskabels (167) ist, der zwischen der vierten beweglichen Rolle (165) und dem dritten Drehelement (153) angeordnet ist.

8. Hinterende-Übertragungsvorrichtung (150) nach Anspruch 7, wobei ein Abschnitt des dritten Übertragungskabels (166), der zwischen dem zweiten Drehelement (152) und der dritten beweglichen Rollen (164) angeordnet ist, parallel zu einem Abschnitt des dritten Übertragungskabels (166) ist, der zwischen der dritten beweglichen Rolle (164) und dem dritten Drehelement (153) angeordnet ist, und parallel zu dem Abschnitt des vierten Übertragungskabels (167) ist, der zwischen der vierten beweglichen Rolle (165) und dem dritten Drehelement (153) angeordnet ist.

9. Medizinische Vorrichtung (120), umfassend die Hinterende-Übertragungsvorrichtung (150) nach einem der Ansprüche 1 bis 8.

10. Chirurgischer Roboter (100), umfassend die medizinische Vorrichtung (120) nach Anspruch 9.

## Revendications

1. Dispositif de transmission arrière (150), comprenant :
un premier élément rotatif (151) ;
un deuxième élément rotatif (152) ;
un troisième élément rotatif (153) ;
un premier ensemble de transmission relié à un premier câble (141) et à un deuxième câble (142) et relié en outre au premier élément rotatif (151) et au troisième élément rotatif (153) ; et
un deuxième ensemble de transmission relié à un troisième câble (143) et à un quatrième câble (144) et relié en outre au deuxième élément rotatif (152) et au troisième élément rotatif (153) ;
dans lequel le premier élément rotatif (151) peut tourner pour tirer l'un parmi le premier câble (141) et le deuxième câble (142) et pour en relâcher un autre parmi le premier câble (141) et le deuxième câble (142) simultanément via le premier ensemble de transmission ;
le deuxième élément rotatif (152) peut tourner pour tirer l'un parmi le troisième câble (143) et le quatrième câble (144) et pour en relâcher un autre parmi le troisième câble (143) et le quatrième câble (144) simultanément via le deuxième ensemble de transmission ; et
le troisième élément rotatif (153) peut tourner pour effectuer, via le premier ensemble de transmission et le deuxième ensemble de transmission :
la traction du premier câble (141) et du deuxième câble (142) tout en relâchant le troisième câble (143) et le quatrième câble (144) ; ou
la traction du troisième câble (143) et du quatrième câble (144) tout en relâchant le premier câble (141) et le deuxième câble (142) ;
**caractérisé en ce que** le premier ensemble de transmission comprend :
une première poulie mobile (156) reliée au premier câble (141) ;
un premier câble de transmission (158) passant sur la première poulie mobile (156), dans lequel le premier câble de transmission (158) présente une extrémité reliée au premier élément rotatif (151) et présente une autre extrémité reliée au troisième élément rotatif (153) ;
une deuxième poulie mobile (157) reliée au deuxième câble (142) ; et
un deuxième câble de transmission (159) passant sur la deuxième poulie mobile (157), dans lequel le deuxième câble de transmission (159) présente une extrémité reliée au premier élément rotatif (151) et présente une autre extrémité reliée au troisième élément rotatif (153) ; dans lequel
le premier câble de transmission (158) s'enroule autour du premier élément rotatif (151) dans une direction opposée à la direction dans laquelle le deuxième câble de transmission (159) s'enroule autour du premier élément rotatif (151), et le premier câble de transmission (158) s'enroule autour du troisième élément rotatif (153) dans une direction identique à une direction dans laquelle le deuxième câble de transmission (159) s'enroule autour du troisième élément rotatif (153) ; et
en rotation du premier élément rotatif (151) ou du troisième élément rotatif (153), la première poulie mobile (156) est entraînée en mouvement par le premier câble de transmission (158) et la deuxième poulie mobile (157) est entraînée en mouvement par le deuxième câble de transmission (159) ;
le deuxième ensemble de transmission comprend :
une troisième poulie mobile (164) reliée au troisième câble (143) ;
un troisième câble de transmission (166) passant sur la troisième poulie mobile (164), dans lequel le troisième câble de transmission (166) présente une extrémité reliée au deuxième élément rotatif (152) et présente une autre extrémité reliée au troisième élément rotatif (153) ;
une quatrième poulie mobile (165) reliée au quatrième câble (144) ; et
un quatrième câble de transmission (167) passant sur la quatrième poulie mobile (165), dans lequel le quatrième câble de transmission (167) présente une extrémité reliée au deuxième élément rotatif (152) et présente une autre extrémité reliée au troisième élément rotatif (153) ; dans lequel
le troisième câble de transmission (166) s'enroule autour du deuxième élément rotatif (152) dans une direction opposée à la direction dans laquelle le quatrième câble de transmission (167) s'enroule autour du deuxième élément rotatif (152), et chacun du troisième câble de transmission (166) et du quatrième câble de transmission (167) s'enroule autour du troisième élément rotatif (153) dans une direction opposée à une direction dans laquelle chacun du premier câble de transmission (158) et du deuxième câble de transmission (159) s'enroule autour du troisième élément rotatif (153) ; et
en rotation du deuxième élément rotatif (152) ou du troisième élément rotatif (153), la troisième poulie mobile (164) est entraînée en mouvement par le troisième câble de transmission (166) et la quatrième poulie mobile (165) est entraînée en mouvement par le quatrième câble de transmission (167).

2. Dispositif de transmission arrière (150) selon la revendication 1, dans lequel le premier élément rotatif (151) peut tourner autour d'un premier axe central (A1) du premier élément rotatif (151), le deuxième élément rotatif (152) peut tourner autour d'un deuxième axe central (A2) du deuxième élément rotatif (152), et le troisième élément rotatif (153) peut tourner autour d'un troisième axe central (A3) du troisième élément rotatif (153) ; et
dans lequel le premier axe central (A1) est parallèle au deuxième axe central (A2) et parallèle au troisième axe central (A3).

3. Dispositif de transmission arrière (150) selon la revendication 1, dans lequel le premier ensemble de transmission comprend en outre une première poulie de guidage (163), et dans lequel
une partie du premier câble de transmission (158) située entre le premier élément rotatif (151) et la première poulie mobile (156) passe sur la première poulie de guidage (163), de sorte qu'une partie du premier câble de transmission (158) située entre la première poulie de guidage (163) et la première poulie mobile (156) est parallèle à une partie du premier câble de transmission (158) située entre la première poulie mobile (156) et le troisième élément rotatif (153).

4. Dispositif de transmission arrière (150) selon la revendication 3, dans lequel une partie du deuxième câble de transmission (159) située entre le premier élément rotatif (151) et la deuxième poulie mobile (157) est parallèle à une partie du deuxième câble de transmission (159) située entre la deuxième poulie mobile (157) et le troisième élément rotatif (153) et parallèle à la partie du premier câble de transmission (158) située entre la première poulie mobile (156) et le troisième élément rotatif (153).

5. Dispositif de transmission arrière (150) selon la revendication 1, dans lequel
la première poulie mobile (156) est reliée de manière pivotante à un premier siège de poulie (181) via un premier arbre (161), et le premier siège de poulie (181) est relié au premier câble (141), et dans lequel la deuxième poulie mobile (157) est reliée de manière pivotante à un deuxième siège de poulie (182) via un deuxième arbre (162), et le deuxième siège de poulie (182) est relié au deuxième câble (142), dans lequel le premier arbre (161) est parallèle au deuxième arbre (162) et parallèle au premier axe central (A1).

6. Dispositif de transmission arrière (150) selon la revendication 5, dans lequel la troisième poulie mobile (164) est reliée de manière pivotante à un troisième siège de poulie (183) via un troisième arbre (168), et le troisième siège de poulie (183) est relié au troisième câble (143), et dans lequel la quatrième poulie mobile (165) est reliée de manière pivotante à un quatrième siège de poulie (184) via un quatrième arbre (169), et le quatrième siège de poulie (184) est relié au quatrième câble (144), dans lequel le troisième arbre (168) est parallèle au quatrième arbre (169) et parallèle au premier axe central (A1).

7. Dispositif de transmission arrière (150) selon l'une quelconque des revendications 5 à 6, dans lequel le deuxième ensemble de transmission inclut en outre une deuxième poulie de guidage (171), et dans lequel
une partie du quatrième câble de transmission (167) située entre le deuxième élément rotatif (152) et la quatrième poulie mobile (165) passe sur la deuxième poulie de guidage (171), de sorte qu'une partie du quatrième câble de transmission (167) située entre la deuxième poulie de guidage (171) et la quatrième poulie mobile (165) est parallèle à une partie du quatrième câble de transmission (167) située entre la quatrième poulie mobile (165) et le troisième élément rotatif (153).

8. Dispositif de transmission arrière (150) selon la revendication 7, dans lequel une partie du troisième câble de transmission (166) située entre le deuxième élément rotatif (152) et la troisième poulie mobile (164) est parallèle à une partie du troisième câble de transmission (166) située entre la troisième poulie mobile (164) et le troisième élément rotatif (153) et parallèle à la partie du quatrième câble de transmission (167) située entre la quatrième poulie mobile (165) et le troisième élément rotatif (153).

9. Dispositif médical (120), comprenant le dispositif de transmission arrière (150) selon l'une quelconque des revendications 1 à 8.

10. Robot chirurgical (100), comprenant le dispositif médical (120) selon la revendication 9.
